# EUROPEAN PATENT SPECIFICATION

(11) **EP 2 962 112 B1**
(45) Date of publication and mention of the grant of the patent: **19.08.2020**
(21) Application number: 14707150.0
(22) Date of filing: 27.02.2014
(51) Int. Cl.: G01N 33/574, G01N 33/68

(54) **METHOD FOR PREDICTING THERAPY EFFICACY USING NUCLEOSOME STRUCTURE BIOMARKERS**
VERFAHREN ZUR VORHERSAGE DER THERAPIEWIRKSAMKEIT MITHILFE VON NUKLEOSOMSTRUKTUR-BIOMARKERN
PROCÉDÉ POUR LA PRÉDICTION D'EFFICACITÉ DE THÉRAPIES METTANT EN UVRE DES BIOMARQUEURS DE STRUCTURE DE NUCLÉOSOMES

(30) Priority: 28.02.2013 GB 201303576; 28.02.2013 US 201361770922 P
(43) Date of publication of application: 06.01.2016
(73) Proprietor: Belgian Volition SPRL, 5032 Isnes (BE)
(72) Inventor: HOLDENRIEDER, Stefan, BE-5032 Isnes (BE)
(74) Representative: Pond, Elizabeth Grace Catherine
(86) International application number: PCT/EP2014/053856
(87) International publication number: WO 2014/131845

(56) References cited:
- WO-A1-2005/019826
- WO-A1-2005/040814
- WO-A2-03/070894
- WO-A2-2006/119264
- WO-A2-2010/120942
- WO-A2-2013/084002
- C. MOHAN ET AL: "Nucleosome: a major immunogen for pathogenic autoantibody-inducing T cells of lupus", JOURNAL OF EXPERIMENTAL MEDICINE, vol. 177, no. 5, 1 May 1993 (1993-05-01), pages 1367-1381, XP055119382, ISSN: 0022-1007, DOI: 10.1084/jem.177.5.1367
- J FÜLLGRABE ET AL: "Histone onco-modifications", ONCOGENE, vol. 30, no. 31, 4 August 2011 (2011-08-04), pages 3391-3403, XP055054538, ISSN: 0950-9232, DOI: 10.1038/onc.2011.121
- GOEBEL G ET AL: "CIRCULATING NUCLEIC ACIDS IN PLASMA OR SERUM (CNAPS) AS PROGNOSTIC AND PREDICTIVE MARKERS IN PATIENTS WITH SOLID NEOPLASIAS", DISEASE MARKERS, WILEY, CHICHESTER, GB, vol. 21, no. 3, 1 January 2005 (2005-01-01), pages 105-120, XP009067705, ISSN: 0278-0240
- MANUYAKORN A ET AL: "Cellular histone modification patterns predict prognosis and treatment response in resectable pancreatic adenocarcinoma: results from RTOG 9704", JOURNAL OF CLINICAL ONCOLOGY, LIPPINCOTT WILLIAMS & WILKINS, USA, vol. 28, no. 8, 10 March 2010 (2010-03-10) , pages 1358-1365, XP002686594, ISSN: 1527-7755, DOI: 10.1200/JCO.2009.24.5639 [retrieved on 2010-02-08]
- V. URBONAVICIUTE ET AL: "High-mobility group box 1 represents a potential marker of disease activity and novel therapeutic target in systemic lupus erythematosus", JOURNAL OF INTERNAL MEDICINE, vol. 270, no. 4, 1 October 2011 (2011-10-01), pages 309-318, XP055053655, ISSN: 0954-6820, DOI: 10.1111/j.1365-2796.2011.02432.x
- HOLDENRIEDER S ET AL: "Nucleosomes in serum of patients with benign and malignant diseases", INTERNATIONAL JOURNAL OF CANCER, JOHN WILEY & SONS, INC, US, vol. 95, no. 2, 20 March 2001 (2001-03-20) , pages 114-120, XP002401433, ISSN: 0020-7136, DOI: 10.1002/1097-0215(20010320)95:2<114::AID-I JC1020>3.0.CO;2-Q
- YVONNE NADINE FAHMUELLER ET AL: "Predictive and prognostic value of circulating nucleosomes and serum biomarkers in patients with metastasized colorectal cancer undergoing Selective Internal Radiation Therapy", BMC CANCER, BIOMED CENTRAL, LONDON, GB, vol. 12, no. 1, 4 January 2012 (2012-01-04), page 5, XP021118120, ISSN: 1471-2407, DOI: 10.1186/1471-2407-12-5
- SACHIN KUMAR ET AL: "Plasma Nucleosome Levels Might Predict Response to Therapy in Patients With Advanced Non-Small-Cell Lung Cancer", CLINICAL LUNG CANCER, vol. 11, no. 1, 1 January 2010 (2010-01-01), pages 36-44, XP055119379, ISSN: 1525-7304, DOI: 10.3816/CLC.2010.n.006
- TERESITA L BRIONES ET AL: "Chemotherapy-induced cognitive impairment is associated with decreases in cell proliferation and histone modifications", BMC NEUROSCIENCE, BIOMED CENTRAL, LONDON, GB, vol. 12, no. 1, 9 December 2011 (2011-12-09), page 124, XP021093306, ISSN: 1471-2202, DOI: 10.1186/1471-2202-12-124
- HERRANZ MICHEL ET AL: "DNA methylation and histone modifications in patients with cancer: potential prognostic and therapeutic targets", METHODS IN MOLECULAR BIOLOGY; [METHODS MOL. BIOL.; ISSN 1064-3745, VOL. 246], HUMANA PRESS, US, vol. 361, 1 January 2007 (2007-01-01), pages 25-62, XP009115201, ISSN: 1064-3745

## Description

### FIELD OF THE INVENTION

The invention relates to a method for predicting the efficacy of therapeutic treatments, particularly neo-adjuvant cancer treatments, using the levels of cell free nucleosome structures.

### BACKGROUND OF THE INVENTION

There are many examples of treatment regimens in many disparate diseases, including cancer, arthritis and mental health disorders where therapies are more successful in some patients than in others. Where this occurs it would be advantageous to patients and health care providers if those patients likely to respond well to a particular treatment and those patients likely to respond less well or poorly could be differentiated in advance of initiating the therapy or treatment. A predictive test would allow patients who are likely to respond well to commence treatment quickly and would prevent ineffective treatment of those patients who are less likely to benefit from the particular therapy or treatment. This means that those patients unlikely to benefit from the treatment can be given alternative treatments quickly. In the case of cancer diseases this may lead to saved lives by ensuring that an effective treatment is given to the patient as early as possible. In addition health care providers benefit through improved patient outcomes at lower cost by earlier treatment and avoidance of use of ineffective and potentially expensive therapies.

In cancer, adjuvant therapy is a secondary treatment given in conjunction with a main treatment. Common examples include chemotherapy and/or radiotherapy as adjuvant therapies to a main surgical treatment.

Neoadjuvant therapy is treatment given before primary therapy. Neoadjuvant therapies include among others chemotherapy, radiotherapy, hormonal based treatment and biological or antibody drug treatment such as trastuzumab for breast cancer. A patient with a cancer may receive neoadjuvant chemotherapy or radiotherapy to shrink a tumor that is inoperable in its current state, so it can be surgically removed.

In addition to shrinking an inoperable tumor for surgery, neoadjuvant therapy is also used for a variety of purposes in the treatment of cancers. In breast cancer for example neoadjuvant therapy may be used to sufficiently shrink a tumor, that could be removed by mastectomy, to allow breast-conserving surgery. Other advantages of neoadjuvant therapy may include targeting of clinically occult micrometastases to minimize the development of a resistant cell population that can form from spontaneous mutations, reduction of microscopic tumor dissemination caused during surgery and prevention of a tumor growth spurt after surgical resection (Liu *et al,* 2010).

However, neoadjuvant therapy also has some disadvantages including the treatment of a greater burden of disease than therapy used after tumor removal, potential promotion of tumor drug resistance, possible loss of tumor staging information, increased risk of surgical or radiation complications and delay in the primary therapy during which time the primary tumor may grow or metastasize (Liu *et a*/, 2010).

In order to maximise the advantages and minimise the disadvantages of neoadjuvant therapies there is a need for a method to predict which patients are likely to benefit most from this treatment and which patients are less likely to benefit and in whom the treatment may be disadvantageous. Surprisingly we have shown that cancer patients most likely to benefit from neoadjuvant therapy are predicted by a simple blood test for nucleosome structures.

The human body comprises several hundred cell types. All of these cell types contain the same genome but widely different phenotypes and different functions in the body. This phenotypic diversity is due to the differential expression of the genome in different cell types. The control of differential gene expression is not entirely understood but the basic mechanisms include gene regulation by a number of interconnected epigenetic signals associated with the gene, including control of the chromatin packing as euchromatin or heterochromatin, control of nucleosome positioning and nuclease accessible sites, methylation of DNA and variation in the structure of the nucleosomes around which the DNA is wrapped.

The nucleosome is the basic unit of chromatin structure and consists of a protein complex of eight highly conserved core histones (comprising of a pair of each of the histones H2A, H2B, H3, and H4). Around this complex is wrapped approximately 146 base pairs of DNA. Another histone, H1 or H5, acts as a linker and is involved in chromatin compaction. The DNA is wound around consecutive nucleosomes in a structure often said to resemble "beads on a string" and this forms the basic structure of open or euchromatin. In compacted or heterochromatin this string is coiled and super coiled into a closed and complex structure (Herranz and Esteller, 2007).

Normal cell turnover in adult humans involves the creation by cell division of some 10¹¹ cells daily and the death of a similar number, mainly by apoptosis. During the process of apoptosis chromatin is broken down into mononucleosomes and oligonucleosomes which are released from the cells. Under normal condition these are removed and the level of circulating nucleosomes found in healthy subjects is low. Elevated levels are found in subjects with a variety of conditions including many cancers, auto-immune diseases, inflammatory conditions, stroke and myocardial infarction (Holdenrieder & Stieber, 2009).

Mononucleosomes and oligonucleosomes can be detected by Enzyme-Linked ImmunoSorbant Assay (ELISA) and several methods have been reported (Salgame *et al*, 1997; Holdenrieder *et al,* 2001; van Nieuwenhuijze *et al,* 2003). These assays typically employ an anti-histone antibody (for example anti-H2B, anti-H3 or anti-H1, H2A, H2B, H3 and H4) as capture antibody and an anti-DNA or anti-H2A-H2B-DNA complex antibody as detection antibody. However, we have found that the results of these assays do not agree with each other. Furthermore, although most circulating DNA in serum or plasma is reported to exist as mono-nucleosomes and oligonucleosomes (Holdenrieder *et al,* 2001), measured levels of nucleosomes and DNA in serum or plasma do not agree well. The correlation coefficient between ELISA results for circulating cell free nucleosome levels and circulating DNA levels as measured by real time PCR (Polymerase Chain Reaction) has been reported to be r=0.531 in serum and r=0.350 in plasma (Holdenrieder *et al*, 2005).

Nucleosome ELISA methods are used in cell culture, primarily as a method to detect apoptosis (Salgame *et al*, 1997; Holdenrieder *et al*, 2001; van Nieuwenhuijze *et al*, 2003), and are also used for the measurement of circulating cell free nucleosomes in serum and plasma (Holdenrieder *et al*, 2001). Cell free serum and plasma nucleosome levels released into the circulation by dying cells have been measured by ELISA methods in studies of a number of different cancers to evaluate their use as a potential biomarker (Holdenrieder *et al*, 2001). Mean circulating nucleosome levels are reported to be high in most, but not all, cancers studied. The highest circulating nucleosome levels were observed in lung cancer subjects. The lowest levels were observed in prostate cancer, which were within the normal range of healthy subjects. However, subjects with malignant tumours are reported to have serum nucleosome concentrations that varied considerably and some subjects with advanced tumour disease were found to have low circulating nucleosome levels, within the range measured for healthy subjects (Holdenrieder *et al*, 2001). Because of this and the variety of non-cancer causes of raised nucleosome levels, circulating nucleosome levels are not used clinically as a biomarker of cancer (Holdenrieder and Stieber, 2009).

The structure of nucleosomes can vary by Post Transcriptional Modification (PTM) of histone proteins and by the inclusion of variant histone proteins. PTM of histone proteins typically occurs on the tails of the eight core histones and common modifications include acetylation, methylation or ubiquitination of lysine residues as well as methylation of arginine residues and phosphorylation of serine residues. Histone modifications are known to be involved in epigenetic regulation of gene expression (Herranz and Esteller, 2007). The structure of the nucleosome can also vary by the inclusion of alternative histone isoforms or variants which are different gene or splice products and have different amino acid sequences. Histone variants can be classed into a number of families which are subdivided into individual types. The nucleotide sequences of a large number of histone variants are known and publicly available for example in the National Human Genome Research Institute NHGRI Histone DataBase (Mariño-Ramírez, L., Levine, K.M., Morales, M., Zhang, S., Moreland, R.T., Baxevanis, A.D., and Landsman, D. The Histone Database: an integrated resource for histones and histone fold-containing proteins. Database Vol.2011. (Submitted) and http://genome.nhgri.nih.gov/histones/complete.shtml), the GenBank (NIH genetic sequence) DataBase, the EMBL Nucleotide Sequence Database and the DNA Data Bank of Japan (DDBJ).

Histone variant and histone modification patterns present in healthy and diseased cells have been shown to differ in numerous (mostly immunohistochemical) studies (Herranz and Esteller, 2007). One disadvantage of immunohistochemical methods for clinical use is that tissue sample collection is invasive involving surgery or biopsy.

In addition to the epigenetic signaling mediated by nucleosome structure and position, control of gene expression in cells is also mediated by the methylation status of DNA (Herranz and Esteller, 2007). It has been known in the art for some time that DNA may be methylated at the 5 position of cytosine nucleotides to form 5-methylcytosine.

The involvement of DNA methylation in cancer was reported as early as 1983 (Feinberg and Vogelstein, 1983). DNA methylation patterns observed in cancer cells differ from those of healthy cells. Repetitive elements, particularly around pericentromeric areas, are reported to be hypomethylated in cancer relative to healthy cells but promoters of specific genes are reported to be hypermethylated in cancer. The balance of these two effects is reported to result in global DNA hypomethylation in cancer cells (Rodriguez-Paredes & Esteller, 2011).

Hypermethylation of certain specific genes can be used as a diagnostic biomarker for cancers. For example a method reported for detection of hypermethylation of the Septin 9 gene by PCR amplification of DNA extracted from plasma was reported to detect 72% of colon cancers with a false positive rate of 10% (Grutzmann *et al,* 2008). The DNA methylation status of specific genes or loci is usually detected by selective bisulphite deamination of cytosine, but not 5-methylcytosine, to uracil, leading to a primary DNA sequence change that can be detected by sequencing or other means (Allen *et al,* 2004).

Global DNA hypomethylation is a hallmark of cancer cells (Esteller 2007 and Hervouet *et al,* 2010). Global DNA methylation can be studied in cells using immunohistochemistry techniques. Alternatively the DNA is extracted from the cells for analysis.

It has been known for many years that, in addition to nucleic acid and histone proteins, chromatin comprises a large number of non-histone proteins bound to its constituent DNA and/or histones (Yoshida and Shimura, 1972). These chromatin associated proteins are of a wide variety of types and have a variety of functions including transcription factors, transcription enhancement factors, transcription repression factors, histone modifying enzymes, DNA damage repair proteins and many more. The study of chromatin bound proteins has been carried out largely by Chromatin ImmunoPrecipitation (ChIP) methods. These methods are well known in the art but are complex, laborious and expensive.

In a typical ChIP method the cellular chromatin is cross-linked so that all the protein and nucleic acid components are covalently attached to each other. The chromatin is then sheared to form a preparation of mononucleosomes and oligonucleosomes. An antibody to the protein of interest is added to the sheared chromatin to immunoprecipitate those chromatin fragments containing the protein. The antibody is normally attached to a solid phase (eg; plastic beads) to facilitate isolation of the chromatin complex containing the protein of interest. The cross-linking is then reversed and the protein is removed by digestion with a proteinase. The DNA associated with the chromatin complex is isolated and analysed to determine the DNA sequence, gene or locus associated with the particular protein binding using any of a variety of techniques including PCR followed by gel electrophoresis, DNA sequencing (ChIP-Seq) or DNA microarrays (ChIP-on-chip).

These ChIP methods reveal the DNA sequences associated with chromatin bound histone proteins. Derivatives of the ChIP method have been developed to facilitate studies of the association of non-histone proteins with histones and nucleosomes including for example Histone Associated Assays (Ricke and Bielinsky, 2005). Many proteins that bind to chromatin are involved in cancer and other disease mechanisms but their abundance in nucleosome adduct form in the circulation has not been previously investigated. Examples include the High Mobility Group Box Protein 1 (HMGB1), the polycomb protein Enhancer of Zeste Homolog 2 (EZH2) and the nuclear receptor group of proteins.

The High Mobility Group of proteins are a component of chromatin present at about 3% of the weight of DNA or histones. They are structural proteins that bind to nucleosomes without any known specificity for the underlying DNA sequence (Gerlitz *et al*; 2009). HMGB1 is an architectural chromosomal protein and a pro-inflammatory mediator. It is involved in cell death, apoptosis and in numerous diseases including various inflammatory and autoimmune conditions, sepsis, meningitis and neurodegeneration. Overexpression of HMGB1 is associated with all of the central hallmarks of cancer (Tang *et al*; 2010). HMGB1 is tightly attached to the chromatin of apoptotic cells. Studies of nucleosome-HMGB1 complexes have shown that these adducts are found in the circulation of subjects suffering from the autoimmune disease Systemic Lupus Erythematosus (SLE) and that the adducts are involved in the development of anti-nuclear antibodies which is a key feature of SLE. .Nucleosomes not attached to HMGB1 do not illicit an immune response. The binding of HMGB1 to nucleosomes in these adducts was demonstrated by immunoprecipitation of nucleosomes with an antibody directed to DNA or histones followed by Western Blot using an anti-HMGB1 antibody to demonstrate the presence of HMGB1 in the immunoprecipitated nucleosomes (Urbonaviciute *et al*; 2008).

HMGB proteins interact with many other proteins known to affect chromatin function and chromatin complexes involving HMGB proteins plus additional proteins have been shown to occur (Gerlitz *et al*; 2009). Thus, in addition to simple nucleosome-protein adducts, nucleosome-protein-complex adducts in which 2 or multiple proteins are associated with nucleosomes occur in chromatin.

EZH2 is a member of the Polycomb-group (PcG) family that form multimeric protein complexes involved in maintaining the transcriptional repressive state of genes. EZH2 is a histone modification enzyme (histone-lysine N-methyltransferase) that methylates the lysine 27 amino acid residue of histone 3 of nucleosomes. This histone modification is associated with chromatin condensation and gene silencing (Cao *et al*; 2002).

Nuclear receptors are molecules that regulate gene expression under the control of hormones or ligands, for example the estrogen receptor (ER) regulates the expression of estrogen dependent genes. Many of these proteins are involved in disease processes, for example ER is involved in the progression of breast cancer and many breast cancer treatments are targeted to ER and/or to prevention of the interaction of ER with its ligand estradiol.

In addition to nucleosome-protein adducts that occur in the cell, there are other nucleosome-protein adducts that may be formed after release of nucleosomes from the cell following cell death. Such nucleosome adducts include the nucleosome-immunoglobulin adducts that are a key feature of SLE.

We now report the prediction of therapeutic efficacy using simple immunoassay methods for the direct estimation of nucleosomes or nucleosomes that contain particular histone modifications or nucleosomes that contain particular histone variants or iso-forms or nucleosomes that contain particular modified nucleotides or nucleosides or protein-nucleosome adducts in biological samples. In particular we have shown that various nucleosome structure biomarkers predict the level of remission achieved with neoadjuvant chemotherapy in cancer.

WO 2005/019826 discloses the diagnosis of disease conditions by contacting cell-free nucleosomes with an antibody that binds specifically with a modified histone protein.

WO 2005/040814 discloses the detection of cancer conditions by determining the presence or amount of post-translational modification of lysine residues within histone sequences.

WO 2010/120942 discloses the identification of altered histone modification patterns selected from H3K4me2, H3K9me2, or H3K18ac for diagnosing and providing a prognosis of therapy for cancer.

Briones, T & Woods, J (2011) BMC Neuroscience, discloses the effects of decreased hippocampal cell proliferation and associated increased histone acetylation in the brains of chemotherapy-treated rats.

Herranz, M & Esteller, M (2007) Methods in Molecular Biology, discloses epigenetic modifications associated with cancer and epigenetic changes and modifications associated with cancer treatments.

### SUMMARY OF THE INVENTION

According to a first aspect of the invention, there is provided a method of predicting responsiveness of a cancer patient to therapeutic treatment comprising the step of detecting the level of a cell free nucleosome moiety selected from a histone modification, histone variant, modified nucleotide, or a nucleosome adducted to another protein, in a blood sample previously obtained from the patient prior to said therapeutic treatment, characterised in that the therapeutic treatment is a neoadjuvant treatment.

According to a second aspect of the invention, there is provided a method of predicting responsiveness of a cancer patient to a neoadjuvant treatment which comprises the steps of:
(i) contacting a blood, serum or plasma sample previously obtained from the subject prior to treatment with a first binding agent which binds to one or more nucleosomes;
(ii) contacting the one or more nucleosomes or sample with a second binding agent which binds to a histone modification, histone variant, modified nucleotide or a protein adducted to a nucleosome;
(iii) detecting or quantifying the binding of said second binding agent to the one or more nucleosomes in the sample; and
(iv) using the detection or quantity of such binding as a parameter for predicting the responsiveness of the patient to neoadjuvant treatment.

According to a third aspect of the invention, there is provided a method of predicting responsiveness of a cancer patient to neoadjuvant treatment which comprises the steps of:
(i) contacting a blood, serum or plasma sample previously obtained from the subject prior to treatment with a first binding agent which binds to one or more of a histone modification, histone variant, modified nucleotide or a protein adducted to a nucleosome;
(ii) contacting the nucleosomes or sample with a second binding agent which binds to nucleosomes;
(iii) detecting or quantifying the binding of said second binding agent to the nucleosomes in the sample; and
(iv) using the detection or quantity of such binding as a parameter for predicting the responsiveness of the patient to neoadjuvant treatment.

### BRIEF DESCRIPTION OF THE FIGURES

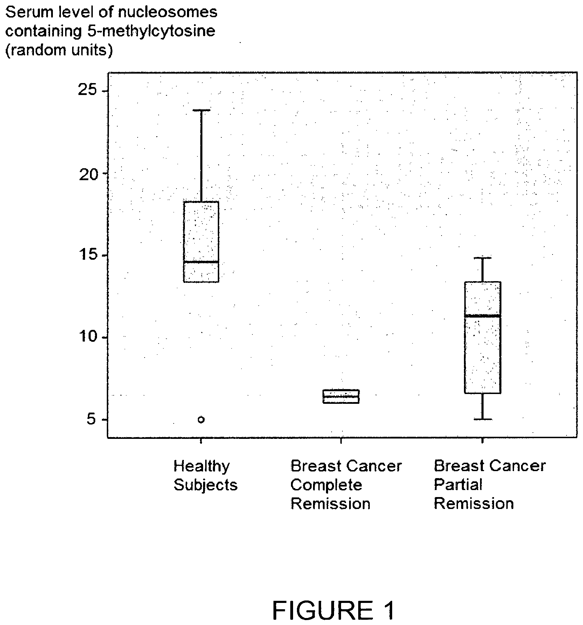

The graphs in Figures 1-4 are standard "box plots" constructed as follows: the lower and upper limits of the data boxes represent the range of values covered by the 2 central quartiles of the data points (i.e. the range between the 25^{th} and the 75^{th} percentile of data points). The lower and upper lines extending from the boxes represent ranges of the lower and upper quartiles of data points (except outliers) where these lie outside the 25^{th} and 75^{th} percentiles. Outlier points, where present, are shown individually as circles.
**Figure 1****:** Measured level of nucleosomes containing the modified nucleotide 5-methylcytosine in serum samples taken from 9 healthy subjects and from 8 diagnosed breast cancer patients prior to any therapy. After blood sampling all the breast cancer patients were treated with a neoadjuvant therapy resulting in complete remission for 2 patients and partial remission for the other 6 patients.

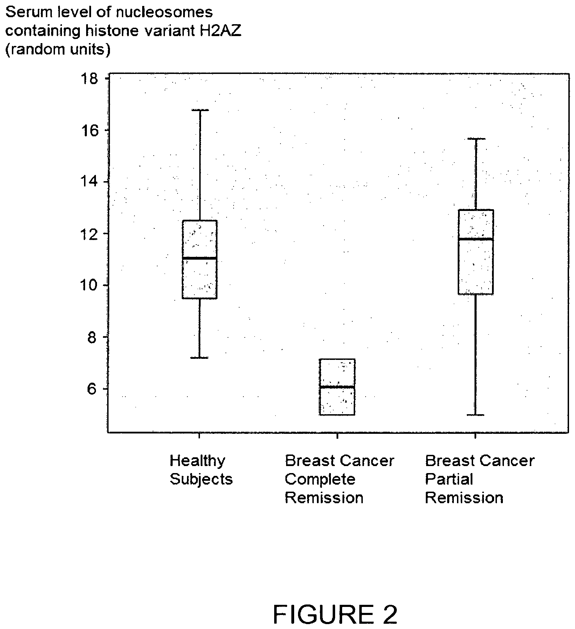

**Figure 2****:** Measured level of nucleosomes containing the histone variant H2AZ in serum samples taken from 9 healthy subjects and from 8 diagnosed breast cancer patients prior to any therapy. After blood sampling all the breast cancer patients were treated with a neoadjuvant therapy resulting in complete remission for 2 patients and partial remission for the other 6 patients.

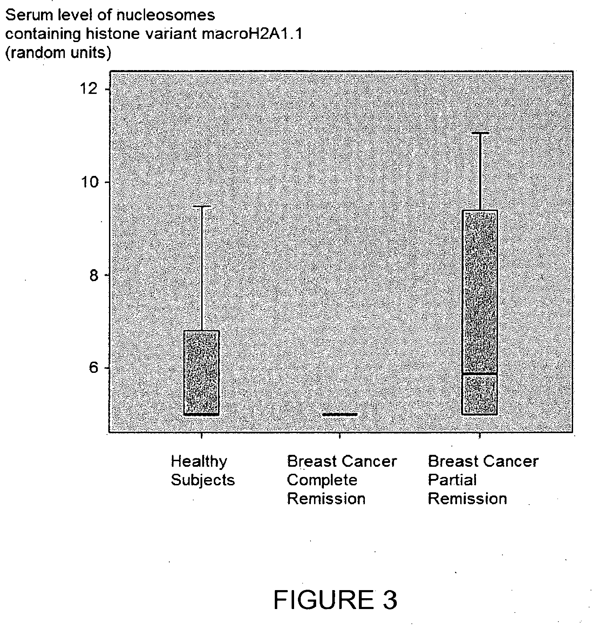

**Figure 3****:** Measured level of nucleosomes containing the histone variant macroH2A1.1 in serum samples taken from 9 healthy subjects and from 8 diagnosed breast cancer patients prior to any therapy. After blood sampling all the breast cancer patients were treated with a neoadjuvant therapy resulting in complete remission for 2 patients and partial remission for the other 6 patients.

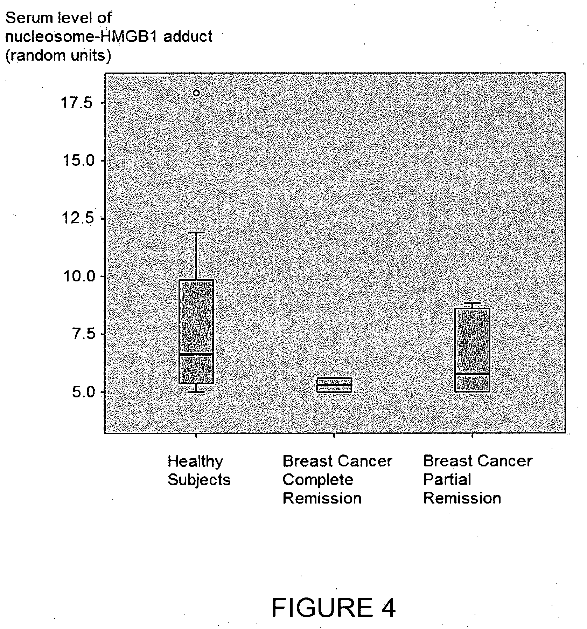

**Figure 4****:** Measured level of nucleosomes-HMGB1 adducts in serum samples taken from 9 healthy subjects and from 8 diagnosed breast cancer patients prior to any therapy. After blood sampling all the breast cancer patients were treated with a neoadjuvant therapy resulting in complete remission for 2 patients and partial remission for the other 6 patients.

### DETAILED DESCRIPTION OF THE INVENTION

According to a first aspect of the invention, there is provided a method of predicting responsiveness of a cancer patient to therapeutic treatment comprising the step of detecting the level of a cell free nucleosome moiety selected from a histone modification, histone variant, modified nucleotide, or a nucleosome adducted to another protein, in a blood sample previously obtained from the patient prior to said therapeutic treatment, characterised in that the therapeutic treatment is a neoadjuvant treatment.

We have shown that the levels of four such nucleosome structures (two nucleosome structures containing particular histone variants, one nucleosome structure containing a particular nucleotide and a nucleosome adduct containing HMGB1) present in the circulation of subjects diagnosed with breast cancer provide predictive information regarding the probable success of neoadjuvant therapy.

We have measured the level of nucleosomes containing the modified nucleotide 5-methylcytosine, nucleosomes containing the histone variant macroH2A1.1, nucleosomes containing the histone variant H2AZ and nucleosome-HMGB1 adducts in the blood of 8 women diagnosed with breast cancer prior to any treatment.

These women received 4 cycles of epirubicine (90 mg/m²) and cyclophosphamide (600 mg/m²), followed by 4 cycles of docetaxel (75 mg/m²) or paclitaxel (175 mg/m²). Herceptin was added to docetaxel in Her2/neu-overexpressing cases. After completion of chemotherapy, definitive surgery, either breast conserving or not, including axillary lymph node surgery, was carried out. Response to neoadjuvant chemotherapy was evaluated by histopathology after surgical tumor resection. Findings were compared with staging investigations before the start of therapy, performed by biopsy, sonography, and mammography. The response to therapy was classified according to RECIST criteria for solid tumors defining 'complete remission' as complete disappearance of all such manifestations of disease, 'partial remission' as reduction of tumor diameter ≥ 30%, 'progression' as tumor increase ≥ 20% or appearance of new tumor manifestations, and 'stable disease' as tumor reduction < 30% or increase < 20% in medical imaging. Pathological complete remission (CR) was defined as the complete lack of histologically visible tumor cells in the final preparation.

The levels of all these nucleosome structures present in the blood prior to neoadjuvant therapy were different in patients in whom remission was partial or complete post therapy. Surprisingly the levels of these nucleosome structures in breast cancer patients prior to treatment is predictive of the post neoadjuvant treatment tumor status. It will be clear to those skilled in the art that the levels of nucleosome structures can be used to predict the patient response to drugs at other stages of disease treatment and not only at the neoadjuvant therapy stage. For example similar tests can be applied to predict the efficacy in patients of similar treatments when used as an adjuvant therapy or as a primary therapy.

In one embodiment, the nucleosome moiety is a nucleosome containing a particular histone modification, histone variant or modified nucleotide.

In one embodiment, the nucleosome moiety is a nucleosome structure containing a modified nucleotide, i.e. a modified nucleosome, such as a nucleosome containing a modified nucleotide (e.g. 5-methylcytosine).

In one embodiment, the nucleosome moiety is a nucleosome structure containing a histone variant. In a further embodiment, the histone variant is macroH2A1.1 or H2AZ.

In one embodiment, the nucleosome moiety is a nucleosome adduct, such as a nucleosome adducted to another protein, for example a nucleosome-HMGB1 adduct.

In one embodiment, said method additionally comprises the step of selecting patients suitable for said neoadjuvant treatment.

In one embodiment of the disclosure, said method additionally comprises the step of administering one or more therapeutic agents.

According to a second aspect of the invention there is provided a method of predicting responsiveness of a cancer patient to neoadjuvant treatment which comprises the steps of:
(i) contacting a blood, serum or plasma sample previously obtained from the subject prior to treatment with a first binding agent which binds to one or more nucleosomes;
(ii) contacting the one or more nucleosomes or sample with a second binding agent which binds to a histone modification, histone variant, modified nucleotide or a protein adducted to a nucleosome;
(iii) detecting or quantifying the binding of said second binding agent to the one or more nucleosomes in the sample; and
(iv) using the detection or quantity of such binding as a parameter for predicting the responsiveness of the subject to neoadjuvant treatment.

It will be clear to those skilled in the art that the binding agent directed to bind to nucleosomes may without limitation be directed to bind any common epitope present in nucleosomes including for example unmodified histone epitopes, core nucleosome epitopes combining more than one histone, DNA or DNA-histone complex epitopes. It will also be clear that the binder to be detected may be selected to be either the antibody directed to the adducted protein or to the nucleosome or a component part of the nucleosome.

According to a third aspect of the invention there is provided a method of predicting responsiveness of a cancer patient to neoadjuvant treatment which comprises the steps of:
(i) contacting a blood, serum or plasma sample previously obtained from the subject prior to treatment with a first binding agent which binds to one or more of a histone modification, histone variant, modified nucleotide or a protein adducted to a nucleosome;
(ii) contacting the nucleosomes or sample with a second binding agent which binds to nucleosomes;
(iii) detecting or quantifying the binding of said second binding agent to the nucleosomes in the sample; and
(iv) using the detection or quantity of such binding as a parameter for predicting the responsiveness of the subject to neoadjuvant treatment.

In one embodiment, the neoadjuvant treatment is a treatment for breast cancer patients.

In one embodiment, the neoadjuvant treatment is a chemotherapy.

In one embodiment, the neoadjuvant treatment comprises administration of a hormone agonist or antagonist.

In one embodiment, the neoadjuvant treatment is a radiotherapy.

In one embodiment, the method additionally comprises the assay of multiple nucleosome structure biomarkers and ratios or other multiple parameters may be used to predict therapy efficacy.

It is well known in the art that cancers may be hormone dependent and require the presence of hormone for growth. It is also well known that nuclear hormones function by nuclear localisation of the receptor bound hormone complex and binding to specific hormone response elements in the genome. The expression of genes associated with the elements is regulated by binding of the receptor bound hormone complex to the genomic response element. In one embodiment the invention provides hormone receptor-nucleosome adduct and hormone-hormone receptor-nucleosome complex adduct biomarkers to characterise the tumour status of a subject in terms of its likely susceptibility to a particular therapy. These adducts may be circulating adducts present in the blood or in another body fluid or may be produced by the digestion of chromatin from a sample of tumour tissue.

It is well known in the art that nuclear hormone receptors regulate gene expression under hormonal or ligand control. For example the estrogen receptor functions by binding its substrate (the steroid hormone estrogen) at the cell surface membrane. Binding is followed by internalisation of the hormone-receptor complex and intra-nuclear localisation where the receptor binds to specific hormone response elements in the genome. The specific gene sequence to which the estrogen receptor binds is known as the Estrogen Response Element (ERE). Expression of genes associated with the ERE may be regulated by the receptor and hence by the presence or level of estrogen in the circulation of a subject. It is also well known in the art that growth of breast cancer is often under estrogen control and such cancer is often termed estrogen dependent. As these tumours overexpress the Estrogen Receptor (ER) they are often termed ER+ tumours.

The growth of estrogen dependent tumours can be slowed or prevented by therapeutic interventions aimed at prevention of estrogen binding to the estrogen receptor and this is a common method of breast cancer treatment. Examples of such treatments include the drug tamoxifen which acts as an antagonist for estrogen in estrogen dependent breast cancer and aromatase inhibitors which slow or prevent estrogen production. However, with time, cancers develop into estrogen independent tumours which will grow even in the absence of estrogen stimulation and require different treatments. The diagnosis of estrogen dependent and independent tumours is currently performed routinely by immunostaining of tumour biopsy tissue to determine the abundance or otherwise of the estrogen receptor in tumour cells. Clinicians may need to retest the estrogen dependency of a tumour repeatedly during the course of tumour treatment to determine whether or not further estrogen dependent treatment is appropriate or whether the subject's treatment regime should be altered to reflect the changing nature of the tumour as the disease progresses. Unfortunately current tests are suboptimal and require a repeated painful biopsy on each occasion the test is performed.

In one embodiment of the invention the detection of estrogen receptor-nucleosome adducts in a blood sample previously obtained from breast cancer patients is used as an indicator of estrogen receptor binding to ERE in the nucleus of tumour cells as an indicator for estrogen dependency of a tumour to aid the selection of appropriate treatment and for predictive prognostic information. This method has the advantages that it is indicative of ERE-estrogen receptor binding in the tumour, rather than a simple indicator of the presence or abundance of estrogen receptor, and that it may be repeated as frequently as desired by a simple blood test without the need for biopsy. We have developed simple ELISA methods for the detection and quantification of nucleosome-ER adducts containing both the ERα and ERβ forms of the receptor.

In a similar manner to estrogen dependent breast cancer, the growth of androgen dependent prostate cancer requires, or is accelerated by androgen. Androgen dependent prostate tumours are similarly treated by methods that prevent androgen binding to the androgen receptor (AR). Androgen dependent prostate tumours may also develop to become androgen independent and hence resistant to treatments including physical or chemical castration by drugs to prevent androgen binding to its receptor. The androgen dependency status of a tumour, and hence its likely response to hormone based and other therapies, may be determined by the level of androgen receptor binding to androgen response elements (ARE) in the genome and this may be determined by the analysis of androgen receptor-nucleosome adduct levels present in the circulation of a subject or in chromatin digests from prostate tissue.

Embodiments of the invention for this purpose include the detection of androgen receptor-nucleosome adducts or androgen-androgen receptor-nucleosome complex adducts in a blood sample previously obtained from a subject or in nucleosomes produced by digestion of chromatin from tumour tissue of a subject.

In one embodiment, the nucleosome biomarker used to predict therapy efficacy is a nucleosome adduct including a pro-inflammatory protein, a High Mobility Group Protein, a polycomb protein, a chromatin modifying enzyme, a nuclear receptor or a hormone. In an alternative embodiment, the nucleosome adduct includes a High Mobility Group Protein, a polycomb protein, a chromatin modifying enzyme, a hormone receptor or a hormone. In a further embodiment, the nucleosome adduct includes a chromatin modifying enzyme, a nuclear receptor or a hormone. In a further embodiment, the High Mobility Group Protein is HMGB1.

In one embodiment, the chromatin modifying enzyme is a histone acetylation, deacetylation, methylation, demethylation phosphorylation, dephosphorylation ubiquitination, deubiquitination sumoylation, desumoylation or DNA methyltransferase enzyme. In an alternative embodiment, the chromatin modifying enzyme is EZH2. It will be clear to those skilled in the art that these types of nucleosome biomarkers will be particularly relevant for the prediction of the efficacy of drug therapies whose mechanism of action involves epigenetic changes. For example assays for nucleosomes containing acetylated histones may be appropriate for prediction of the efficacy of Histone DeAcetylation Complex (HDAC) inhibitor drugs or assays for nucleosomes containing methylated histones may be appropriate for prediction of the efficacy of Histone Methyl Transferase (HMT) inhibitor drugs or assays for nucleosomes containing modified nucleotides may be appropriate for prediction of the efficacy of DNA MethylTransferase (DNMT) inhibitor drugs.

In one embodiment, the binding agent used is an antibody, an antibody fragment or an aptamer. In a further embodiment, the binding agent used is an antibody.

In one embodiment, the sample is blood or serum or plasma. It will be clear to those skilled in the art that the detection of nucleosome adducts in blood or serum or plasma has the advantage of being a minimally invasive method that does not require biopsy.

In one embodiment, the nucleosome adduct is detected or measured as one of a panel of measurements.

According to a further aspect of the disclosure, there is provided a kit for the detection of a nucleosome structure which comprises a ligand or binder specific for the nucleosome structure or component part thereof, or a structural/shape mimic of the nucleosome structure or component part thereof, together with instructions for use of the kit in accordance with any one of the methods defined herein.

HMGB1 is a damage associated molecular pattern (DAMP) protein associated with cell death, apoptosis and numerous diseases including various inflammatory and autoimmune conditions, sepsis, meningitis, neurodegeneration, SLE and cancer (Tang *et al;* 2010). Elevated expression of HMGB1 occurs in many cancers and is thought to be associated with invasion and metastases (Sims *et al,* 2010). Elevated levels of HMGB1 also occur in the blood of cancer patients as well as in a variety of other conditions (Stoetzer *et al,* 2012). Circulating HMGB1 can be measured by ELISA but such measurements are not used in routine clinical practice because circulating HMGB1 occurs in bound and free forms and the Western immunoblot methods currently available to distinguish these are not suitable for routine use. Therefore there is a need for a reliable method to distinguish between free HMGB1 and HMGB1 complexes (Urbonaviciute and Voll, 2011). An important class of circulating HMGB1 complexes is HMGB1-nucleosome adducts and one embodiment of the present invention is directed to the detection of HMGB1-nucleosome adducts and other HMG-nucleosome adducts. We have shown that HMG-nucleosome adducts can be measured in the blood of cancer patients using a rapid and simple ELISA method.

HMGB1 is tightly attached to the chromatin of apoptotic cells. Studies of nucleosome-HMGB1 complexes have shown that these adducts are found in the circulation of subjects suffering from the autoimmune disease SLE and that the adducts are involved in the development of anti-nuclear antibodies which is a key feature of SLE. The presence of these adducts in the circulation has not been used for clinical diagnostic purposes because the Western blot methods used for their detection are expensive, slow and laborious and not suitable for routine clinical use. The present invention overcomes these shortcomings.

EZH2 is a chromatin modification enzyme (histone-lysine N-methyltransferase) that methylates the lysine 27 amino acid residue of histone 3 of nucleosomes leading to chromatin condensation and gene silencing (Cao *et al*; 2002). This protein is known to bind chromatin in the nucleus of living cells. Surprisingly we have shown that EZH2 remains bound to nucleosomes after cell death and mononucleosome-EZH2 and oligonucleosome-EZH2 adducts can be detected in the serum of cancer subjects using the novel ELISA methods of the present invention.

It is known that chromatin modifying enzymes are involved in cancer (Fullgrabe *et al,* 2011) and inhibition of the activity of these enzymes through the use of targeted drugs is a major form of cancer therapy. These drugs include for example and without limitation Histone Deacetylation Complex inhibitors (HDACi), Histone Methyl Transferase inhibitors (HMTi) and DNA Methyl Transferase inhibitors (DNMTi). Whilst the presence of HMGB1 adducts in the circulation is known to be pathological and associated with anti-nuclear antibodies, the finding that chromatin modifying enzyme-nucleosome adducts are present in the circulation has not previously been reported. Assays for chromatin modifying enzyme-nucleosome adducts have multiple uses in cancer including for example in the assessment of cancer disease states and in the determination of the efficacy of chromatin modifying enzyme inhibitor drugs, for example to determine if the level of circulating chromatin modifying enzyme-nucleosome adduct is altered by treatment with particular drugs. The method of the invention may be used to determine circulating chromatin modifying enzyme-nucleosome adduct levels for a wide variety of disease diagnostic purposes including disease detection, monitoring, prognosis, differential diagnosis and choice of treatment regimes. We have shown that nucleosome adducts containing the HMT enzyme EZH2 can be detected in the circulation of cancer patients. It will be clear to those skilled in the art that the method of the invention may be applied to other chromatin modifying enzymes including the before mentioned HDAC and DNMT enzymes as well as many other enzymes including for example enzymes for histone acetylation, demethylation, phosphorylation, dephosphorylation, ubiquitination, deubiquitination, sumoylation and desumoylation.

Nuclear receptors exert their gene regulatory effects in the nucleus under ligand hormone control. Examples include the steroid hormone receptors, thyroid receptor, glucocorticoid receptor and retinoic acid and vitamin D receptor. These receptors are involved in a variety of cancer and other disease mechanisms. Some examples include the involvement of the Retinoic Acid Receptor (RAR) in leukaemia, the Estrogen Receptor (ER) in breast cancer and endometriosis, the Androgen Receptor (AR) in prostate cancer and the Thyroid Hormone Receptor in thyroid disease and cancer.

According to one aspect of the disclosure there is provided a double antibody, immunometric or sandwich immunoassay method for detecting and measuring cell free nucleosome structures in a sample for therapy efficacy prediction. One embodiment of the disclosure is an immunoassay which comprises the steps of:
(i) contacting a sample which may contain nucleosomes with a first antibody or other binder which binds to nucleosomes or a component thereof;
(ii) contacting the nucleosomes or sample with a second antibody or other binder which binds to a histone modification, histone variant, modified nucleotide or a protein that may be present as a nucleosome-protein adduct;
(iii) detecting and/or quantifying the binding of said second antibody or other binder; and
(iv) using the presence or degree of such binding as predictive of therapy efficacy.

According to another aspect of the disclosure there is provided a method for detecting and measuring cell free nucleosome structures in a sample by an immunometric immunoassay which comprises the steps of:
(i) contacting a sample which may contain nucleosomes with a first antibody or other binder which binds to a histone modification, histone variant, modified nucleotide or a protein that may be present as a nucleosome-protein adduct;
(ii) contacting the nucleosomes or sample with a second antibody or other binder which binds to nucleosomes or a component thereof;
(iii) detecting and/or quantifying the binding of said second antibody or other binder to nucleosomes sample with a second antibody or other binder which binds to nucleosomes or a component thereof in the sample; and
(iv) using the presence or degree of such binding to predict the efficacy of a therapy.

It will be clear to those skilled in the art that the antibody or other binder used to bind nucleosomes or a component thereof in stage (i) of the first aspect of the disclosure above and stage (ii) of the second aspect of the disclosure above may be an antibody (or other binder) directed against intact nucleosomes or against any component part of a nucleosome including without limitation against a histone, a histone variant, a histone modification, a nucleotide, a modified nucleotide or other part of the DNA component of a nucleosome.

It will be clear to those skilled in the art that the methods of the invention described include a variety of embodiments including biosensor type assays and label-free assays of the type marketed for example by ForteBio Incorporated of USA.

According to a further aspect of the disclosure there is provided a method for identifying a nucleosome structure biomarker for predicting responsiveness of a subject to therapeutic treatment which comprises the steps of:
(i) detecting or measuring a nucleosome structure in a body fluid sample taken from the subject prior to treatment;
(ii) treating the subject with said treatment; and
(iii) using the difference between the levels detected in patients in whom the treatment was successful or less successful to identify whether a nucleosome structure is useful as a biomarker for the selection of patients for the said treatment.

According to a further aspect of the disclosure, there is provided a biomarker identified by the method as defined herein.

A further aspect of the disclosure provides ligands or binders, such as naturally occurring or chemically synthesised compounds, capable of specific binding to the biomarker. A ligand or binder according to the disclosure may comprise a peptide, an antibody or a fragment thereof, or a synthetic ligand such as a plastic antibody, or an aptamer or oligonucleotide, capable of specific binding to the biomarker. The antibody can be a monoclonal antibody or a fragment thereof capable of specific binding to the biomarker. A ligand according to the disclosure may be labeled with a detectable marker, such as a luminescent, fluorescent, enzyme or radioactive marker; alternatively or additionally a ligand according to the disclosure may be labelled with an affinity tag, e.g. a biotin, avidin, streptavidin or His (e.g. hexa-His) tag. Alternatively ligand binding may be determined using a label-free technology for example that of ForteBio Inc.

A biosensor according to the disclosure may comprise the biomarker or a structural/shape mimic thereof capable of specific binding to an antibody against the biomarker. Also disclosed is an array comprising a ligand or mimic as described herein.

Also provided by the invention is the use of one or more ligands as described herein, which may be naturally occurring or chemically synthesised, and is suitably a peptide, antibody or fragment thereof, aptamer or oligonucleotide, or the use of a biosensor of the disclosure, or an array of the disclosure, or a kit of the disclosure to detect and/or quantify the biomarker. In these uses, the detection and/or quantification can be performed on a biological sample as defined herein.

Diagnostic kits are disclosed for performing methods of the invention. Such kits will suitably comprise a ligand according to the invention, for detection and/or quantification of the biomarker, and/or a biosensor, and/or an array as described herein, optionally together with instructions for use of the kit.

A further aspect of the disclosure is a kit for predicting the efficacy of a therapy, comprising a biosensor capable of detecting and/or quantifying one or more of the biomarkers as defined herein.

Biomarkers are essential targets for discovery of novel targets and drug molecules that retard or halt progression of the disorder. As the level of the biomarker is indicative of disorder and of drug response, the biomarker is useful for identification of novel therapeutic compounds in *in vitro* and/or *in vivo* assays. Biomarkers can be employed in methods for screening for compounds that modulate the activity of the biomarker.

Thus, in a further aspect of the disclosure, there is provided the use of a binder or ligand, as described, which can be a peptide, antibody or fragment thereof or aptamer or oligonucleotide according to the invention; or the use of a biosensor according to the disclosure, or an array according to the disclosure; or a kit according to the disclosure, to identify a substance capable of promoting and/or of suppressing the generation of the biomarker.

Also there is disclosed a method of identifying a substance capable of promoting or suppressing the generation of the biomarker in a subject, comprising administering a test substance to a subject animal and detecting and/or quantifying the level of the biomarker present in a test sample from the subject.

The term "biomarker" means a distinctive biological or biologically derived indicator of a process, event, or condition. Biomarkers can be used in methods of diagnosis, e.g. clinical screening, and prognosis assessment and in monitoring the results of therapy, identifying subjects most likely to respond to a particular therapeutic treatment, drug screening and development. Biomarkers and uses thereof are valuable for identification of new drug treatments and for discovery of new targets for drug treatment.

The terms "detecting" and "diagnosing" as used herein encompass identification, confirmation, and/or characterisation of a disease state. Methods of detecting, monitoring, diagnosis and of predicting responsiveness according to the invention are useful to confirm the existence of a disease, to monitor development of the disease by assessing onset and progression, or to assess amelioration or regression of the disease. Methods of detecting, monitoring, diagnosis and of predicting responsiveness are also useful in methods for assessment of clinical screening, prognosis, choice of therapy, evaluation of therapeutic benefit, i.e. for drug screening and drug development.

Efficient diagnosis and monitoring methods provide very powerful "subject solutions" with the potential for improved prognosis, by establishing the correct diagnosis, allowing rapid identification of the most appropriate treatment (thus lessening unnecessary exposure to harmful drug side effects), and reducing relapse rates.

In one embodiment, said biomarker is released from the cells of a tumour. Thus, according to a further aspect of the disclosure there is provided a method for the detection of a tumour growth which comprises the steps of (i) measuring a biomarker in a biological sample that is associated with or released from the cells of a tumour and (ii) demonstrating that the level of said biomarker is associated with the size, stage, aggressiveness or dissemination of the tumour.

It is known that increased cell turnover, cell death and apoptosis lead to increased circulatory levels of cell free nucleosomes (Holdenrieder *etal,* 2001). Circulating cell free nucleosomes level is a non-specific indicator and occurs in a variety of conditions including inflammatory diseases, a large variety of benign and malignant conditions, autoimmune diseases, as well as following trauma or ischaemia (Holdenrieder *et al* 2001).

Immunoassays for use in the invention include immunometric assays employing enzyme detection methods (for example ELISA), fluorescence labelled immunometric assays, time-resolved fluorescence labelled immunometric assays, chemiluminescent immunometric assays, immunoturbidimetric assays, particulate labelled immunometric assays and immunoradiometric assays and competitive immunoassay methods including labelled antigen and labelled antibody competitive immunoassay methods with a variety of label types including radioactive, enzyme, fluorescent, time-resolved fluorescent and particulate labels. All of said immunoassay methods are well known in the art, see for example Salgame *et al,* 1997 and van Nieuwenhuijze *et al,* 2003.

Biological samples that may be tested in a method of the invention include whole blood, blood serum or plasma. Further examples of biological samples according to the disclosure include cerebrospinal fluid (CSF), menstrual blood, endometrial fluid, urine, saliva, or other bodily fluid (stool, tear fluid, synovial fluid, sputum), breath, e.g. as condensed breath, or an extract or purification therefrom, or dilution thereof. Biological samples also include specimens from a live subject, or taken post-mortem. The samples can be prepared, for example where appropriate diluted or concentrated, and stored in the usual manner.

The method of the invention may be repeated on multiple occasions. This embodiment of the disclosure provides the advantage of allowing the detection results to be monitored over a time period. Such an arrangement will provide the benefit of monitoring or assessing the efficacy of treatment of a disease state. Such monitoring methods of the disclosure can be used to monitor onset, progression, stabilisation, amelioration, relapse and/or remission.

Thus, the disclosure also provides a method of monitoring efficacy of a therapy for a disease state in a subject, suspected of having such a disease, comprising detecting and/or quantifying the biomarker present in a biological sample from said subject. In monitoring methods, test samples may be taken on two or more occasions. The method may further comprise comparing the level of the biomarker(s) present in the test sample with one or more control(s). The method of the disclosure may comprise detecting a change in the nature or amount of the biomarker(s) in test samples taken on different occasions.

Thus, according to a further aspect of the disclosure, there is provided a method for monitoring efficacy of therapy for a disease state in a human or animal subject, comprising:
(a) quantifying the amount of the biomarker as defined herein; and
(b) comparing the amount of said biomarker in a test sample with the amount present in one or more control(s) and/or one or more previous test sample(s) taken at an earlier time from the same test subject.

A change in the level of the biomarker in the test sample relative to the level in a previous test sample taken earlier from the same test subject may be indicative of a beneficial effect, e.g. stabilisation or improvement, of said therapy on the disorder or suspected disorder. Furthermore, once treatment has been completed, the method of the invention may be periodically repeated in order to monitor for the recurrence of a disease.

Methods for monitoring efficacy of a therapy can be used to monitor the therapeutic effectiveness of existing therapies and new therapies in human subjects and in non-human animals (e.g. in animal models). These monitoring methods can be incorporated into screens for new drug substances and combinations of substances.

In a further embodiment of the disclosure the monitoring of more rapid changes due to fast acting therapies may be conducted at shorter intervals of hours or days.

According to a further aspect of the disclosure, there is provided a method for identifying a biomarker for detecting the presence of a disease state. The term "identifying" as used herein means confirming the presence of the biomarker present in the biological sample. Quantifying the amount of the biomarker present in a sample may include determining the concentration of the biomarker present in the sample. Identifying and/or quantifying may be performed directly on the sample, or indirectly on an extract therefrom, or on a dilution thereof.

In alternative aspects of the disclosure, the presence of the biomarker is assessed by detecting and/or quantifying antibody or fragments thereof capable of specific binding to the biomarker that are generated by the subject's body in response to the biomarker and thus are present in a biological sample from a subject having a disease state.

Identifying and/or quantifying can be performed by any method suitable to identify the presence and/or amount of a specific protein in a biological sample from a subject or a purification or extract of a biological sample or a dilution thereof. In methods of the invention, quantifying may be performed by measuring the concentration of the biomarker in the sample or samples. Biological samples that may be tested in a method of the invention include those as defined hereinbefore. The samples can be prepared, for example where appropriate diluted or concentrated, and stored in the usual manner.

Identification and/or quantification of biomarkers may be performed by detection of the biomarker or of a fragment thereof, e.g. a fragment with C-terminal truncation, or with N-terminal truncation. Fragments are suitably greater than 4 amino acids in length, for example 5, 6, 7, 8, 9, 10, 11, 12, 13, 14, 15, 16, 17, 18, 19, or 20 amino acids in length. It is noted in particular that peptides of the same or related sequence to that of histone tails are particularly useful fragments of histone proteins.

The biomarker may be directly detected, e.g. by SELDI or MALDI-TOF. Alternatively, the biomarker may be detected directly or indirectly via interaction with a ligand or ligands such as an antibody or a biomarker-binding fragment thereof, or other peptide, or ligand, e.g. aptamer, or oligonucleotide, capable of specifically binding the biomarker. The ligand or binder may possess a detectable label, such as a luminescent, fluorescent or radioactive label, and/or an affinity tag.

For example, detecting and/or quantifying can be performed by one or more method(s) selected from the group consisting of: SELDI (-TOF), MALDI (-TOF), a 1-D gel-based analysis, a 2-D gel-based analysis, Mass spec (MS), reverse phase (RP) LC, size permeation (gel filtration), ion exchange, affinity, HPLC, UPLC and other LC or LC MS-based techniques. Appropriate LC MS techniques include ICAT® (Applied Biosystems, CA, USA), or iTRAQ® (Applied Biosystems, CA, USA). Liquid chromatography (e.g. high pressure liquid chromatography (HPLC) or low pressure liquid chromatography (LPLC)), thin-layer chromatography, NMR (nuclear magnetic resonance) spectroscopy could also be used.

Methods of predicting responsiveness according to the invention may comprise analysing a sample by SELDI TOF or MALDI TOF to detect the presence or level of the biomarker. These methods are also suitable for clinical screening, prognosis, monitoring the results of therapy, identifying subjects most likely to respond to a particular therapeutic treatment, for drug screening and development, and identification of new targets for drug treatment.

Identifying and/or quantifying the analyte biomarkers may be performed using an immunological method, involving an antibody, or a fragment thereof capable of specific binding to the biomarker. Suitable immunological methods include sandwich immunoassays, such as sandwich ELISA, in which the detection of the analyte biomarkers is performed using two antibodies which recognize different epitopes on a analyte biomarker; radioimmunoassays (RIA), direct, indirect or competitive enzyme linked immunosorbent assays (ELISA), enzyme immunoassays (EIA), Fluorescence immunoassays (FIA), western blotting, immunoprecipitation and any particle-based immunoassay (e.g. using gold, silver, or latex particles, magnetic particles, or Q-dots). Immunological methods may be performed, for example, in microtitre plate or strip format.

In one embodiment of the disclosure, one or more of the biomarkers may be replaced by a molecule, or a measurable fragment of the molecule, found upstream or downstream of the biomarker in a biological pathway.

The identification of key biomarkers specific to a disease is central to integration of diagnostic procedures and therapeutic regimes. Using predictive biomarkers appropriate diagnostic tools such as biosensors can be developed; accordingly, in methods and uses of the invention, identifying and quantifying can be performed using a biosensor, microanalytical system, microengineered system, microseparation system, immunochromatography system or other suitable analytical devices. The biosensor may incorporate an immunological method for detection of the biomarker(s), electrical, thermal, magnetic, optical (e.g. hologram) or acoustic technologies. Using such biosensors, it is possible to detect the target biomarker(s) at the anticipated concentrations found in biological samples.

As used herein, the term "biosensor" means anything capable of detecting the presence of the biomarker. Examples of biosensors are described herein.

Biosensors according to the disclosure may comprise a ligand binder or ligands, as described herein, capable of specific binding to the biomarker. Such biosensors are useful in detecting and/or quantifying a biomarker.

The biomarker(s) can be detected using a biosensor incorporating technologies based on "smart" holograms, or high frequency acoustic systems, such systems are particularly amenable to "bar code" or array configurations.

In smart hologram sensors (Smart Holograms Ltd, Cambridge, UK), a holographic image is stored in a thin polymer film that is sensitised to react specifically with the biomarker. On exposure, the biomarker reacts with the polymer leading to an alteration in the image displayed by the hologram. The test result read-out can be a change in the optical brightness, image, colour and/or position of the image. For qualitative and semi-quantitative applications, a sensor hologram can be read by eye, thus removing the need for detection equipment. A simple colour sensor can be used to read the signal when quantitative measurements are required. Opacity or colour of the sample does not interfere with operation of the sensor. The format of the sensor allows multiplexing for simultaneous detection of several substances. Reversible and irreversible sensors can be designed to meet different requirements, and continuous monitoring of a particular biomarker of interest is feasible.

Suitably, biosensors for detection of one or more biomarkers combine biomolecular recognition with appropriate means to convert detection of the presence, or quantitation, of the biomarker in the sample into a signal. Biosensors can be adapted for "alternate site" diagnostic testing, e.g. in the ward, outsubjects' department, surgery, home, field and workplace.

Biosensors to detect one or more biomarkers include acoustic, plasmon resonance, holographic, Bio-Layer Interferometry (BLI) and microengineered sensors. Imprinted recognition elements, thin film transistor technology, magnetic acoustic resonator devices and other novel acousto-electrical systems may be employed in biosensors for detection of the one or more biomarkers.

Methods involving identification and/or quantification of one or more biomarkers can be performed on bench-top instruments, or can be incorporated onto disposable, diagnostic or monitoring platforms that can be used in a non-laboratory environment, e.g. in the physician's office or at the subject's bedside. Suitable biosensors for performing methods of the invention include "credit" cards with optical or acoustic readers. Biosensors can be configured to allow the data collected to be electronically transmitted to the physician for interpretation and thus can form the basis for e-medicine.

Diagnostic kits for the diagnosis and monitoring of the presence of a disease state are described herein. In one embodiment of the disclosure, the kits additionally contain a biosensor capable of identifying and/or quantifying a biomarker. Suitably a kit according to the disclosure may contain one or more components selected from the group: a ligand binder, or ligands, specific for the biomarker or a structural/shape mimic of the biomarker, one or more controls, one or more reagents and one or more consumables; optionally together with instructions for use of the kit in accordance with any of the methods defined herein.

The identification of biomarkers for a disease state permits integration of diagnostic procedures and therapeutic regimes. Detection of a biomarker can be used to screen subjects prior to their participation in clinical trials. The biomarkers provide the means to indicate therapeutic response, failure to respond, unfavourable side-effect profile, degree of medication compliance and achievement of adequate serum drug levels. The biomarkers may be used to provide warning of adverse drug response. Biomarkers are useful in development of personalized therapies, as assessment of response can be used to fine-tune dosage, minimise the number of prescribed medications, reduce the delay in attaining effective therapy and avoid adverse drug reactions. Thus by monitoring a biomarker of the invention, subject care can be tailored precisely to match the needs determined by the disorder and the pharmacogenomic profile of the subject, the biomarker can thus be used to titrate the optimal dose, predict a positive therapeutic response and identify those subjects at high risk of severe side effects.

Biomarker-based tests provide a first line assessment of 'new' subjects, and provide objective measures for accurate and rapid diagnosis, not achievable using the current measures.

Furthermore, diagnostic biomarker tests are useful to identify family members or subjects with mild or asymptomatic disease or who may be at high risk of developing symptomatic disease. This permits initiation of appropriate therapy, or preventive measures, e.g. managing risk factors. These approaches are recognised to improve outcome and may prevent overt onset of the disorder.

Biomarker monitoring methods, biosensors and kits are also vital as subject monitoring tools, to enable the physician to determine whether relapse is due to worsening of the disorder. If pharmacological treatment is assessed to be inadequate, then therapy can be reinstated or increased; a change in therapy can be given if appropriate. As the biomarkers are sensitive to the state of the disorder, they provide an indication of the impact of drug therapy.

The invention will now be illustrated with reference to the following non-limiting examples.

### EXAMPLE 1

Serum samples were taken from 9 healthy subjects and 8 subjects diagnosed with breast cancer prior to any therapy. The breast cancer patients were then treated with neoadjuvant therapy prior to surgery for tumour resection. The neoadjuvant therapy resulted in complete remission for 2 of the breast cancer patients but no remission or only partial remission in the other 6 patients. Remission was ascertained on surgery and by histological findings.

The sera taken from subjects were assayed in duplicate for the level of nucleosomes containing the modified nucleotide 5-methylcytosine, nucleosomes containing the histone variant macroH2A1.1, nucleosomes containing the histone variant H2AZ and nucleosome-HMGB1 adducts using 4 ELISA methods as described below. Neat commercially available horse serum produced for use in tissue culture was also assayed as a negative control sample containing no nucleosomes or nucleosome adducts.

The ELISA methods used a solid phase anti-histone capture antibody that binds intact nucleosomes in combination with the appropriate biotinylated anti-5-methylcytosine, or anti-macroH2A1.1, or anti-H2AZ or anti-HMGB1 detection antibody as follows: A solution of anti-histone antibody in 0.1M phosphate buffer pH 7.4 was added to microtitre wells (100 µL/well) and incubated to coat the wells with capture antibody. Excess anti-histone antibody was decanted. A solution of bovine serum albumin (20g/L) was added to the wells (200 µL/well) and incubated to block excess protein binding sites on the wells. Excess bovine serum albumin solution was decanted and the wells were washed three times with wash buffer (200 µL/well, 0.05M TRIS/HCI buffer pH 7.5 containing 1% Tween 20). Serum sample (10 µL/well) and assay buffer (50 µL/well, 0.05M TRIS/HCI pH 7.5 containing 0.9% NaCl, 0.05% sodium deoxycholate and 1% Nonidet P40 substitute) were added to the wells incubated overnight at 4°C. The serum and assay buffer mixture was decanted and the wells were washed three times with wash buffer (200 µL/well). A solution of the appropriate biotinylated detection antibody was added (50 µL/well) and incubated. Excess detection antibody was decanted and the wells were again washed three times with wash buffer (200 µL/well). A solution containing a streptavidin-horse radish peroxidase conjugate was added (50 µL/well) and incubated at room temperature. Excess conjugate was decanted and the wells were again washed three times with wash buffer (200 µL/well). A coloured substrate solution (100 µL/well, 2,2'-Azinobis [3-ethylbenzothiazoline-6-sulfonic acid]-diammonium salt) was added and incubated for 20 minutes at room temperature with mild agitation. The optical density (OD) of the wells was measured at a wavelength of 405nm using a standard microtitre plate reader. A dose response curve of increasing colour with increasing nucleosome structure concentration was observed with a low background signal observed in the absence of nucleosome adduct (horse serum).

The observed level of nucleosomes containing the modified nucleotide 5-methylcytosine, nucleosomes containing the histone variant macroH2A1.1, nucleosomes containing the histone variant H2AZ and nucleosome-HMGB1 adducts in women with breast cancer prior to neoadjuvant therapy were different in those women from whom the treatment resulted in complete remission compared to those women for whom treatment resulted in no remission or partial remission. Thus it is clear that the observed nucleosome structure levels are predictive of the probability of therapeutic outcome for the treatment and can be used in advance to those patients for whom the therapy is most likely to result in complete remission. The results are shown in Figures 1-4.

### REFERENCES

Allen et al, A simple method for estimating global DNA methylation using bisulfite PCR of repetitive DNA elements. Nucleic Acids Research: 32(3) e38DOI: 10.1093/nar/gnh032, 2004
Bawden *et al,* Detection of histone modification in cell-free nucleosomes. WO 2005/019826, 2005
Cao et al, Role of Histone H3 Lysine 27 Methylation in Polycomb-Group Silencing SCIENCE 298, 1039-1043, 2002
Dai etal, Detection of Post-translational Modifications on Native Intact Nucleosomes by ELISA. http://www.jove.com/details.php?id=2593 doi: 10.3791/2593. J Vis Exp. 50 (2011).
Esteller, Cancer epigenomics: DNA methylomes and histone-modification maps Nature Reviews Genetics: 8, 286-298, 2007
Feinberg and Vogelstein, Hypomethylation distinguishes genes of some human cancers from their normal counterparts. Nature: 301, 89-92, 1983
Fullgrabe et al, Histone onco-modifications. Oncogene: 30, 3391-3403, 2011
Gerlitz et al, The dynamics of HMG protein-chromatin interactions in living cells. Biochem Cell Biol, 87, 127-137, 2009
Grutzmann et al, Sensitive Detection of Colorectal Cancer in Peripheral Blood by Septin 9 DNA Methylation Assay. PLoS ONE 3(11): e3759. doi:10.1371 /journal.pone.0003759, 2008
Herranz and Esteller, DNA methylation and histone modifications in subjects with cancer: potential prognostic and therapeutic targets. Methods Mol Biol.361:25-62, 2007
Hervouet et al, Disruption of Dnmt1/PCNA/UHRF1 Interactions Promotes Tumorigenesis from Human and Mice Glial Cells PLoS ONE 5(6): e11333. doi:10.1371/journal.pone.0011333, 2010
Holdenrieder et al, Nucleosomes in serum of subjects with benign and malignant diseases. Int. J. Cancer (Pred. Oncol.): 95, 114-120, 2001
*Holdenrieder et al, Nucleosomes in Serum as a Marker for Cell Death. Clin Chem Lab Med; 39(7), 596-605, 2001
Holdenrieder et al, Cell-Free DNA in Serum and Plasma: Comparison of ELISA and Quantitative PCR. Clinical Chemistry: 51(8), 1544-1546, 2005
Holdenrieder and Stieber, Clinical use of circulating nucleosomes. Critical Reviews in Clinical Laboratory Sciences; 46(1): 1-24, 2009
Liu et al, Neoadjuvant therapy for breast cancer. Journal of Surgical Oncology; 101, 283-291, 2010
Ricke and Bielinsky, Easy detection of chromatin binding proteins by the histone association assay. Biol Proced Online; 7(1), 60-69, 2005
Rodriguez-Paredes and Esteller, Cancer epigenetics reaches mainstream oncology. Nature Medicine: 17(3), 330-339, 2011
Salgame et al, An ELISA for detection of apoptosis. Nucleic Acids Research, 25(3), 680-681, 1997
Sims etal, HMGB1 and RAGE in inflammation and cancer. Annu. Rev. Immunol. 28, 367-388, 2010
Stoetzer et al, Circulating nucleosomes and biomarkers of immunogenic cell death as predictive and prognostic markers in cancer patients undergoing cytotoxic therapy. Expert Opin Biol Ther. 12(Suppl. 1): S217-S224, 2012
Tang et al, High-mobility Group Box 1 [HMGB1] and Cancer. Biochim Biophys Acta. 1799(1-2) 131, 2010
Urbonaviciute etal, Induction of inflammatory and immune responses by HMGB1 - nucleosome complexes: implications for the pathogenesis of SLE. J Exp Med, 205(13), 3007-3018, 2008
Urbonaviciute and Voll, High-mobility group box 1 represents a potential marker of disease and novel therapeutic target in systemic lupus erythematosus. J Internal Medicine, 270, 309-318, 2011
van Nieuwenhuijze et al, Time between onset of apoptosis and release of nucleosomes from apoptotic cells: putative implications for sysytemic lupus erythematosus. Ann Rheum Dis; 62: 10-14, 2003
Yoshida and Shimura, Isolation of nonhistone chromosomal protein from calf thymus. Biochimica et Biophysica Acta (BBA) - Protein Structure; 263(3), 690-695, 1972

## Claims

1. An in vitro method of predicting responsiveness of a cancer patient to therapeutic treatment comprising the step of detecting the level of a cell free nucleosome moiety selected from a histone modification, histone variant, modified nucleotide, or a nucleosome adducted to another protein, in a blood sample previously obtained from the patient prior to said therapeutic treatment, **characterised in that** the therapeutic treatment is a neoadjuvant treatment.

2. The method as defined in claim 1, wherein said method additionally comprises the step of selecting patients suitable for said neoadjuvant treatment.

3. The method as defined in claim 1, wherein the modified nucleotide is 5-methylcytosine.

4. The method as defined in claim 1, wherein the histone variant is selected from macroH2A1.1 or H2AZ.

5. The method as defined in claim 1, wherein the nucleosome moiety is a nucleosome-HMGB1 adduct.

6. The method as defined in any one of claims 1 to 5 wherein the neoadjuvant treatment is a treatment for breast cancer patients.

7. The method as defined in any one of claims 1 to 6 wherein the neoadjuvant treatment is a chemotherapy, a hormone agonist or antagonist, and/or a radiotherapy.

8. An in vitro method of predicting responsiveness of a cancer patient to a neoadjuvant treatment which comprises the steps of:
(i) contacting a blood, serum or plasma sample previously obtained from the patient prior to treatment with a first binding agent which binds to one or more nucleosomes;
(ii) contacting the one or more nucleosomes or sample with a second binding agent which binds to a histone modification, histone variant, modified nucleotide or a protein adducted to a nucleosome;
(iii) detecting or quantifying the binding of said second binding agent to the one or more nucleosomes in the sample; and
(iv) using the detection or quantity of such binding as a parameter for predicting the responsiveness of the patient to neoadjuvant treatment.

9. An in vitro method of predicting responsiveness of a cancer patient to neoadjuvant treatment which comprises the steps of:
(i) contacting a blood, serum or plasma sample previously obtained from the patient prior to treatment with a first binding agent which binds to one or more of a histone modification, histone variant, modified nucleotide or a protein adducted to a nucleosome;
(ii) contacting the nucleosomes or sample with a second binding agent which binds to nucleosomes;
(iii) detecting or quantifying the binding of said second binding agent to the nucleosomes in the sample; and
(iv) using the detection or quantity of such binding as a parameter for predicting the responsiveness of the patient to neoadjuvant treatment.

10. The method as defined in claim 8 or claim 9 wherein said method additionally comprises the step of selecting patients suitable for said neoadjuvant treatment.

11. A method according to any one of claims 8 to 10 , wherein the nucleosome structure is detected or measured as one of a panel of measurements.

## Patentansprüche

1. In-vitro-Verfahren zur Vorhersage eines Ansprechens eines Krebspatienten auf eine therapeutische Behandlung, den Schritt umfassend, in dem in einer Blutprobe, die dem Patienten vor der therapeutischen Behandlung entnommen wird, der Anteil an einer zellfreien Nukleosomengruppe erfasst wird, der ausgewählt ist aus einer Histonmodifikation, einer Histonvariante, einem modifizierten Nukleotid oder einem Nukleosom, das an einem weiteren Protein angelagert ist, **dadurch gekennzeichnet, dass** die therapeutische Behandlung eine neoadjuvante Behandlung ist.

2. Verfahren nach Anspruch 1, wobei das Verfahren zusätzlich den Schritt umfasst, in dem geeignete Patienten für die neoadjuvante Behandlung ausgewählt werden.

3. Verfahren nach Anspruch 1, wobei das modifizierte Nukleotid 5-Methylcytosin ist.

4. Verfahren nach Anspruch 1, wobei die Histonvariante ausgewählt ist aus macroH2A1.1 oder H2AZ.

5. Verfahren nach Anspruch 1, wobei die Nukleosomengruppe ein Nukleosomen-HMGB1-Addukt ist.

6. Verfahren nach einem der Ansprüche 1 bis 5, wobei die neoadjuvante Behandlung eine Behandlung für Brustkrebspatienten ist.

7. Verfahren nach einem der Ansprüche 1 bis 6, wobei die neoadjuvante Behandlung eine Chemotherapie, eine Therapie mit Hormonagonisten oder Hormonantagonisten und/oder eine Strahlentherapie ist.

8. In-vitro-Verfahren zur Vorhersage eines Ansprechens eines Krebspatienten auf eine neoadjuvante Behandlung, die folgenden Schritte umfassend:
(i) Inkontaktbringen einer Blut-, Serum- oder Plasmaprobe, die dem Patienten vorher entnommen wird, mit einem ersten Bindemittel, das an ein oder mehrere Nukleosomen bindet, vor der Behandlung;
(ii) Inkontaktbringen des einen oder der mehreren Nukleosomen oder Proben mit einem zweiten Bindemittel, das an eine Histonmodifikation, eine Histonvariante, ein modifiziertes Nukleotid oder ein Protein, das an ein Nukleosom angelagert ist, bindet;
(iii) Erfassen oder Quantifizieren des Bindens des zweiten Bindemittels an das eine oder die mehreren Nukleosomen in der Probe; und
(iv) Verwenden der Erfassung oder der Quantität solchen Bindens als Parameter zur Vorhersage des Ansprechens des Patienten auf eine neoadjuvante Behandlung.

9. In-vitro-Verfahren zur Vorhersage eines Ansprechens eines Krebspatienten auf eine neoadjuvante Behandlung, die folgenden Schritte umfassend:
(i) Inkontaktbringen einer Blut-, Serum- oder Plasmaprobe, die dem Patienten vorher entnommen wird, mit einem ersten Bindemittel, das an eines oder mehrere von einer Histonmodifikation, einer Histonvariante, einem modifizierten Nukleotid oder einem Protein, das an ein Nukleosom angelagert ist, bindet;
(ii) Inkontaktbringen der Nukleosomen oder Proben mit einem zweiten Bindemittel, das an Nukleosomen bindet;
(iii) Erfassen oder Quantifizieren des Bindens des zweiten Bindemittels die Nukleosomen in der Probe; und
(iv) Verwenden der Erfassung oder der Quantität solchen Bindens als Parameter zur Vorhersage des Ansprechens des Patienten auf eine neoadjuvante Behandlung.

10. Verfahren nach Anspruch 8 oder Anspruch 9, wobei das Verfahren zusätzlich den Schritt umfasst, in dem geeignete Patienten für die neoadjuvante Behandlung ausgewählt werden.

11. Verfahren nach einem der Ansprüche 8 bis 10, wobei die Nukleosomstruktur als eine aus einer Gruppe von Messungen erfasst oder gemessen wird.

## Revendications

1. Procédé in vitro de prédiction de la sensibilité d'un patient souffrant d'un cancer à un traitement thérapeutique comprenant l'étape de détection du taux d'un fragment de nucléosome acellulaire sélectionné parmi une modification d'histone, un variant d'histone, un nucléotide modifié ou un nucléosome adduit à une autre protéine, dans un échantillon de sang préalablement prélevé sur le patient avant ledit traitement thérapeutique, **caractérisé en ce que** le traitement thérapeutique est un traitement néoadjuvant.

2. Procédé selon la revendication 1, dans lequel ledit procédé comprend en outre l'étape de sélection de patients appropriés pour ledit traitement néoadjuvant.

3. Procédé selon la revendication 1, dans lequel le nucléotide modifié est la 5-méthylcytosine.

4. Procédé selon la revendication 1, dans lequel le variant d'histone est sélectionné parmi macroH2A1.1 ou H2AZ.

5. Procédé selon la revendication 1, dans lequel le fragment de nucléosome est un adduit nucléosome-HMGB1.

6. Procédé selon l'une quelconque des revendications 1 à 5, dans lequel le traitement néoadjuvant est un traitement pour les patients souffrant d'un cancer du sein.

7. Procédé selon l'une quelconque des revendications 1 à 6, dans lequel le traitement néoadjuvant est une chimiothérapie, un agoniste ou antagoniste d'hormone, et/ou une radiothérapie.

8. Procédé in vitro de prédiction de la sensibilité d'un patient souffrant d'un cancer à un traitement néoadjuvant qui comprend les étapes consistant à :
(i) mettre en contact un échantillon de sang, de sérum ou de plasma préalablement prélevé sur le patient avant le traitement avec un premier agent de liaison qui se lie à un ou plusieurs nucléosomes ;
(ii) mettre en contact les un ou plusieurs nucléosomes ou l'échantillon avec un second agent de liaison qui se lie à une modification d'histone, un variant d'histone, un nucléotide modifié ou une protéine adduite au nucléosome ;
(iii) détecter ou quantifier la liaison dudit second agent de liaison aux un ou plusieurs nucléosomes dans l'échantillon ; et
(iv) utiliser la détection ou la quantité d'une telle liaison comme paramètre pour prédire la sensibilité du patient au traitement néoadjuvant.

9. Procédé in vitro de prédiction de la sensibilité d'un patient souffrant d'un cancer à un traitement néoadjuvant qui comprend les étapes consistant à :
(i) mettre en contact un échantillon de sang, de sérum ou de plasma prélevé préalablement sur le patient avant le traitement avec un premier agent de liaison qui se lie à un ou plusieurs parmi une modification d'histone, un variant d'histone, un nucléotide modifié ou une protéine adduite à un nucléosome ;
(ii) mettre en contact les nucléosomes ou l'échantillon avec un second agent de liaison qui se lie aux nucléosomes ;
(iii) détecter ou quantifier la liaison dudit second agent de liaison aux nucléosomes dans l'échantillon ; et
(iv) utiliser la détection ou la quantité d'une telle liaison comme paramètre pour prédire la sensibilité du patient au traitement néoadjuvant

10. Procédé selon la revendication 8 ou la revendication 9, dans lequel ledit procédé comprend en outre l'étape de sélection de patients appropriés pour ledit traitement néoadjuvant.

11. Procédé selon l'une quelconque des revendications 8 à 10, dans lequel la structure de nucléosome est détectée ou mesurée comme l'une d'un panel de mesures.
